# EUROPÄISCHE PATENTSCHRIFT

(11) **EP 3 878 417 B1**
(45) Veröffentlichungstag und Bekanntmachung des Hinweises auf die Patenterteilung: **06.09.2023**
(21) Anmeldenummer: 21157720.0
(22) Anmeldetag: 17.02.2021
(51) Int. Cl.: A61F 9/008

(54) **VORRICHTUNG ZUR ABLATIONSBEARBEITUNG VON OPHTHALMOLOGISCHEM IMPLANTATIONSMATERIAL**
DEVICE FOR ABLATION TREATMENT OF OPHTHALMOLOGIC IMPLANTATION MATERIAL
DISPOSITIF DE TRAITEMENT PAR ABLATION DE LA MATIÈRE D'IMPLANT OPHTALMOLOGIQUE

(30) Priorität: 26.02.2020 CH 2312020
(43) Veröffentlichungstag der Anmeldung: 15.09.2021
(73) Patentinhaber: Ziemer Ophthalmic Systems AG, 2562 Port (CH)
(72) Erfinder: RATHJEN, Christian, 28197 Bremen (DE)
(74) Vertreter: Rentsch Partner AG

(56) Entgegenhaltungen:
- EP-A1- 0 209 992
- US-A1- 2003 153 904
- US-A1- 2009 160 075
- US-A1- 2013 023 863
- US-A1- 2015 335 477

## Beschreibung

### Technisches Gebiet

Die vorliegende Erfindung betrifft eine Vorrichtung zur Ablationsbearbeitung von ophthalmologischem Implantationsmaterial. Die vorliegende Erfindung betrifft insbesondere eine Vorrichtung zur Ablationsbearbeitung von ophthalmologischem Implantationsmaterial, welches durch wasserhaltiges Grundmaterial gebildet ist.

### Stand der Technik

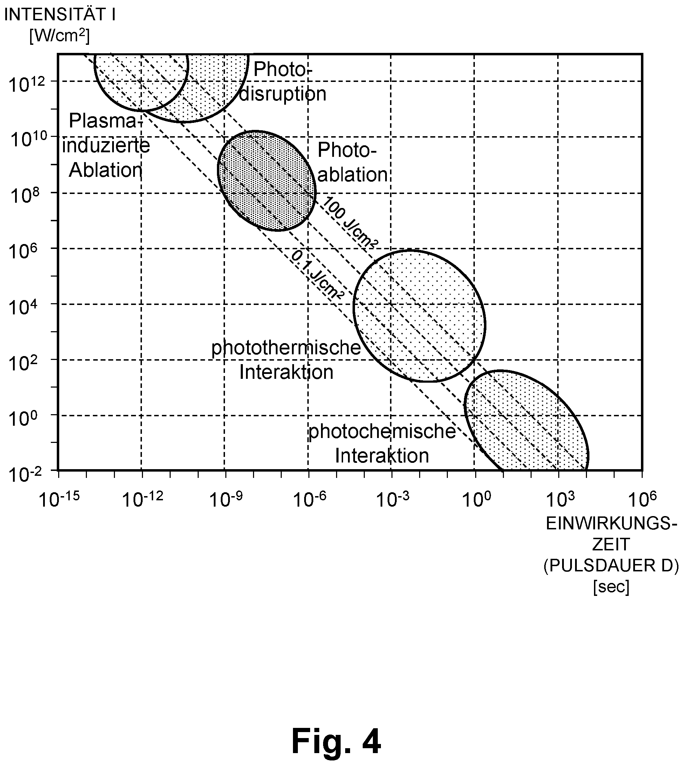

Laserablationsverfahren und dazu geeignete Laservorrichtungen, die über Absorption von Laserenergie an der Oberfläche Materialabtrag bewirken, sind bekannt. Hierbei kommen unterschiedlichste Verfahren, Laserwellenlängen und Plusdauern zum Einsatz. Die Figur 4 basiert auf einer Veröffentlichung von Michael Kaschke, Karl-Heinz Donnerhacke, and Michael Stefan Rill, "Optical Devices in Ophthalmology and Optometry: Technology, Design Principles, and Clinical Applications", 22. Januar 2014, Wiley-VCH Verlag GmbH & Co. KGaA, und illustriert die Abgrenzung von Ablationsverfahren zu anderen Lasermaterialbearbeitungsverfahren. Wie aus der Figur 4 ersichtlich ist, zeichnen sich Photoablationsverfahren durch einen Parameterbereich PA aus, in welchem mit gepulsten Laserstrahlen mit Intensitäten im Bereich von ca. 10⁷ W/cm² bis ca. 10¹⁰ W/cm² und Einwirkungszeiten respektive Pulsdauern von ca. 10⁻⁹ Sekunden bis ca. 10⁻⁶ Sekunden gearbeitet wird. Wie in der Figur 4 zudem ersichtlich ist, entspricht dies Bestrahlungsenergien im Bereich von ca. 10⁻¹ J/cm² bis ca. 10³ J/cm². In der Medizintechnik werden Ablationsverfahren mit Wellenlängen im Ultraviolett (UV) und im Infrarot (IR) Bereich ausgeführt, weil in diesen Wellenlängenbereichen eine erhöhte Absorption in Wasser vorliegt.

Ablationsverfahren werden für die Bearbeitung verschiedener Materialen verwendet, z.B. für die Bearbeitung von Hartmaterialien wie Zähne oder Diamanten. Für die Ablationsbearbeitung von ophthalmologischem Gewebematerial in der refraktiven Chirurgie werden Excimer Laser verwendet, da deren kurze Wellenlängen durch Wasser und Proteine stark absorbiert werden und ein tiefes Eindringen ins Augengewebe und damit verbundene unbeabsichtigte Gewebeschäden ausbleiben. Die Wellenlängen (193nm) von ArF (Argon Fluorid) Excimer-Lasern werden um ein Mehrfaches (ca. 20-mal) stärker von Wasser absorbiert, als Wellenlängen, die über 200nm liegen. Damit einhergehend hat die Luft-Feuchtigkeit im zu bearbeitenden Gewebe und ein eventueller Feuchtigkeitsfilm auf dem Gewebe bei der Bearbeitung mit ArF Excimer-Lasern einen vergleichsweise wesentlich stärkeren Einfluss auf Ablationsabtrag als bei längeren Wellenlängen. Deshalb wird in der refraktiven Chirurgie die Hornhaut (Cornea) vor der Ablation getrocknet, insbesondere durch Abtupfen, und die Luftfeuchtigkeit im Operationssaal definiert eingestellt, um eine zu starke Feuchtigkeit der Cornea und damit einhergehende verstärkte Absorption im Wasser zu vermeiden.

US 2015/0335477 beschreibt ein System für die ophthalmologische Chirurgie mit einer Laserquelle mit einem Laserstrahl mit Laserpulsen, die eine Wellenlänge zwischen 320nm und 430nm haben und Pulsdauern zwischen 1ps und 100ns. Mittels eines optischen Systems wird der Laserstrahl auf fokale Spots im Augengewebe gerichtet und gemäss einem Scannmuster geführt. US 201 5/0335477 erwähnt auch Ausführungsvarianten, in denen eine Spitzenleistungsdichte (peak power density) im Bereich von 20GW/cm² bis 2000GW/cm² verwendet wird, um Augengewebe mit Licht mit einer Wellenlänge von 355nm zu schneiden.

EP 0209992 beschreibt einen Scanning Laser für die Ablation von Cornea-Gewebe mittels Ultraviolett-Strahlung. Im Zusammenhang mit Cornea-Transplantations-Prozeduren verweist EP 0209992 auch auf die Bearbeitung von Cornea-Implantaten, die eingesetzt werden. Gemäss EP 0209992 werden bevorzugt Laser eingesetzt, die im Ultraviollet-Bereich strahlen eingesetzt, wobei auch ein Krypton-Fluorid Laser mit einer Wellenlänge von 248nm angegebenen wird. Im Zusammenhang mit einem Excimer Laser mit einem Argon-Fluorid Laser, welcher eine Wellenlänge von 193nm aufweist, beschreibt EP 0209992 eine Ausführung mit einer maximalen Pulsenergie von 200 mJ und einer Spotgrösse von 0.5mm x 0.5mm.

US 2013/023863 beschreibt ein weiteres System für die Ablation von Cornea-Gewebe mittels Ultraviolett-Strahlung, wobei Wellenlängen im Bereich von 193nm bis 205nm angegeben werden und auch auf mögliche Anwendungen für die Bearbeitung von Cornea-Implantaten erwähnt wird.

### Darstellung der Erfindung

Es ist eine Aufgabe der vorliegenden Erfindung eine Vorrichtung zur Ablationsbearbeitung von ophthalmologischem Implantationsmaterial vorzuschlagen, welche zumindest einige Nachteile der bekannten Systeme nicht aufweist.

Gemäss der vorliegenden Erfindung werden diese Ziele durch die Merkmale des unabhängigen Anspruchs erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass zur Ablationsbearbeitung von ophthalmologischem Implantationsmaterial, welches durch wasserhaltiges Grundmaterial gebildet ist, eine Vorrichtung bereitgestellt wird, welche eine Laserquelle umfasst, die eingerichtet ist, einen gepulsten Laserstrahl mit einer Bearbeitungswellenlänge im Ultraviolett-Wellenbereich zu erzeugen, wobei die Bearbeitungswellenlänge grösser als 193nm ist und im Grundmaterial des Implantationsmaterials einen höheren Absorptionsgrad des Laserstrahls bewirkt als der Absorptionsgrad des Laserstrahls im Wasser des Implantationsmaterials. Die Vorrichtung umfasst zudem eine Projektionsoptik, die eingerichtet ist, den gepulsten Laserstrahl auf eine Oberfläche des Implantationsmaterials zu strahlen, und in einem Bearbeitungsbereich mit Laserpulsen des Laserstrahls, welche Laserpulse eine photoablationbewirkende Kombination von Pulsdauer und Intensität aufweisen, eine Interaktion mit dem Implantationsmaterial zur Ablation des Implantationsmaterials auszulösen. Die Vorrichtung umfasst weiter eine Scannervorrichtung, die eingerichtet ist, eine Bewegung des Bearbeitungsbereichs zur Ablationsbearbeitung gemäss einem Bearbeitungsmuster auszuführen.

In einer Ausführungsvariante ist die Laserquelle eingerichtet, den gepulsten Laserstrahl mit einer Bearbeitungswellenlänge in einem Wellenlängenbereich zu erzeugen, welcher im tieferen Wellenlängenbereich durch einen maximal zu erreichenden Absorptionsgrad von 10⁻²/cm des Laserstrahls im Wasser des Implantationsmaterials begrenzt ist und welcher im höheren Wellenlängenbereich durch einen minimal zu erreichenden Absorptionsgrad von 10⁰/cm des Laserstrahls im Grundmaterial des Implantationsmaterials begrenzt ist.

Erfindungsgemäss ist die Laserquelle eingerichtet, den gepulsten Laserstrahl mit einer Bearbeitungswellenlänge in einem Wellenlängenbereich von 200nm bis 250nm zu erzeugen.

In einer Ausführungsvariante liegt die Pulsdauer der Laserpulse in einem Pulsdauerbereich von 10⁻¹⁰ Sekunden bis 10⁻⁵ Sekunden, insbesondere in einem Pulsdauerbereich von 10⁻⁹ Sekunden bis 10⁻⁶ Sekunden.

Erfindungsgemäss liegt die Intensität der Laserpulse in einem Intensitätsbereich von 10⁶ W/cm² bis 10¹¹ W/cm² liegt, insbesondere in einem Intensitätsbereich von 10⁷ W/cm² bis 10¹⁰ W/cm².

In einer Ausführungsvariante sind die Laserquelle und die Projektionsoptik eingerichtet, den gepulsten Laserstrahl mit einer Fluenz im Fluenzbereich von 10⁶ W/cm² und 10¹⁰ W/cm² auf die Oberfläche des Implantationsmaterials zu strahlen.

In einer Ausführungsvariante umfasst die Vorrichtung einen Luftbefeuchter, einen Luftfeuchtigkeitssensor und eine mit dem Luftbefeuchter und dem Luftfeuchtigkeitssensor verbundene Steuereinrichtung. Dabei umfasst die Steuereinrichtung einen elektronischen Schaltkreis, der eingerichtet ist, den Luftbefeuchter abhängig von einem vom Luftfeuchtigkeitssensor in einem an das Implantationsmaterial angrenzenden Umgebungsbereich gemessenen Luftfeuchtigkeitswert derart zu steuern, dass ein vorgegebener Mindestluftfeuchtigkeitswert eingehalten wird.

In einer Ausführungsvariante ist der elektronische Schaltkreis eingerichtet, den Luftbefeuchter derart zu steuern, dass ein Mindestluftfeuchtigkeitswert von 90% relativer Feuchtigkeit eingehalten wird, insbesondere ein Mindestluftfeuchtigkeitswert von 95% relativer Feuchtigkeit.

In einer Ausführungsvariante ist die Scannervorrichtung eingerichtet, die Bewegung des Bearbeitungsbereichs zur Ablationsbearbeitung gemäss einem Bearbeitungsmuster zur Erzeugung einer Lentikeloberfläche auszuführen.

In einer Ausführungsvariante umfasst die Scannervorrichtung mindestens einen beweglichen Spiegel, der eingerichtet ist, den gepulsten Laserstrahl zur Bewegung des Bearbeitungsbereichs gemäss dem Bearbeitungsmuster abzulenken.

In einer Ausführungsvariante ist die Scannervorrichtung der Projektionsoptik nachgeschaltet angeordnet.

In einer Ausführungsvariante umfasst die Scannervorrichtung mindestens einen Antrieb, der eingerichtet ist, die Projektionsoptik zu verschieben, um die Bewegung des Bearbeitungsbereichs gemäss dem Bearbeitungsmuster auszuführen.

In einer Ausführungsvariante umfasst die Scannervorrichtung mindestens einen Antrieb, der eingerichtet ist, einen Materialträger zu verschieben, auf dem das Implantationsmaterial angebracht ist, um die Bewegung des Bearbeitungsbereichs gemäss dem Bearbeitungsmuster auszuführen.

### Kurze Beschreibung der Zeichnungen

Nachfolgend wird eine Ausführung der vorliegenden Erfindung anhand eines Beispiels beschrieben. Das Beispiel der Ausführung wird durch die folgenden beigelegten Figuren illustriert:
- Figur 1:: zeigt schematisch im Querschnitt ein Blockdiagramm mit Ausführungsvarianten einer Vorrichtung zur Ablationsbearbeitung von ophthalmologischem Implantationsmaterial.
- Figur 2:: zeigt schematisch im Querschnitt ein Blockdiagramm mit weiteren Ausführungsvarianten einer Vorrichtung zur Ablationsbearbeitung von ophthalmologischem Implantationsmaterial.
- Figur 3:: zeigt eine Grafik, welche abhängig von der Wellenlänge des Laserstrahls die Absorption von Laserlicht in Wasser und in das Grundmaterial der Cornea bildendem Protein illustriert.
- Figur 4:: zeigte eine Grafik, welche die Parameterbereiche verschiedener Materialbearbeitungsverfahren mittels Laser darstellt.

### Wege zur Ausführung der Erfindung

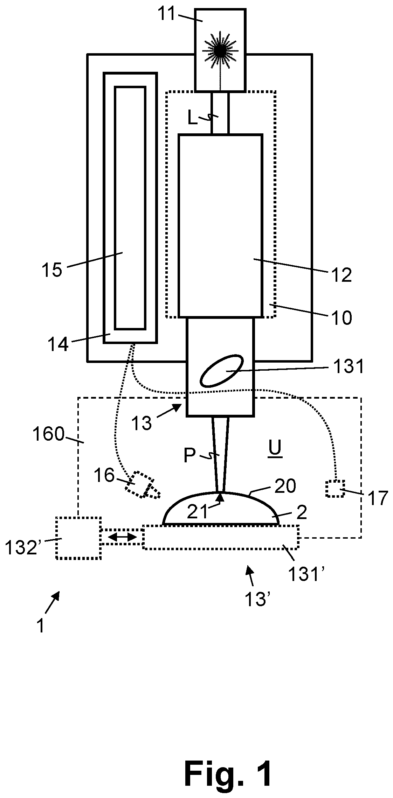

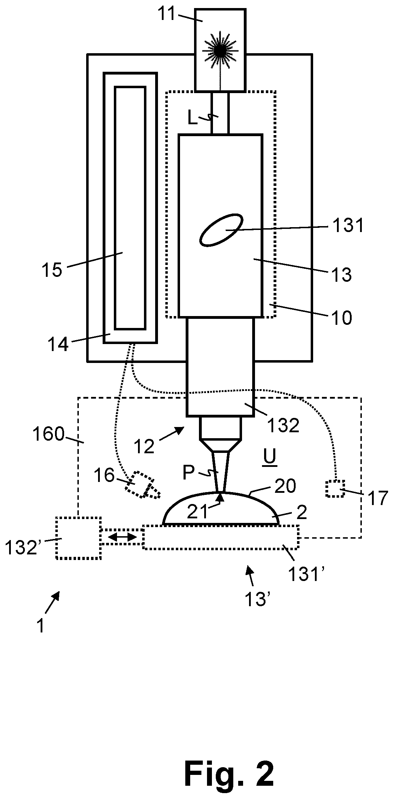

In den Figuren 1 und 2 bezieht sich das Bezugszeichen 1 jeweils auf eine Vorrichtung zur Ablationsbearbeitung, insbesondere auf eine Vorrichtung zur Ablationsbearbeitung von ophthalmologischem Implantationsmaterial 2, insbesondere von wasserhaltigem ophthalmologischen Implantationsmaterial 2. Das ophthalmologische Implantationsmaterial 2 umfasst somit Grundmaterial und Wasser. Das ophthalmologische Implantationsmaterial 2 umfasst natürliches Spendergewebe, z.B. menschliches Hornhautgewebe (Cornea) mit Protein(en) als Grundmaterial, oder synthetisches Gewebe, z.B. Hydrogele, die Wasser enthaltende Polymere umfassen.

Wie in den Figuren 1 und 2 schematisch dargestellt ist, umfasst die Vorrichtung 1 eine Laserquelle 11, eine Projektionsoptik 12, eine Scannervorrichtung 13, 13' sowie eine Steuereinrichtung 14 mit einem elektronischen Schaltkreis 15. Die Steuereinrichtung 14 respektive der elektronische Schaltkreis 15 ist mit der Laserquelle 11, der Projektionsoptik 12 und der Scannervorrichtung 13, 13' für deren Steuerung über Signal- und/oder Steuerleitungen verbunden.

Die Laserquelle 11 ist eingerichtet einen gepulsten Laserstrahl L mit einer Bearbeitungswellenlänge λ im Ultraviolett-Wellenbereich zu erzeugen, wie später detaillierter erläutert und definiert wird. Die Projektionsoptik 12 ist eingerichtet, den gepulsten Laserstrahl L auf eine Oberfläche 20 des Implantationsmaterials 2 zu strahlen und in einem Bearbeitungsbereich 21 mit Laserpulsen P des Laserstrahls L eine Interaktion mit dem Implantationsmaterial 2 zur Ablation des Implantationsmaterials 2 auszulösen. Dazu weisen die von der Laserquelle 11 erzeugten und von der Projektionsoptik 12 auf die Oberfläche 20 des Implantationsmaterials 2 gestrahlten Laserpulse P eine Kombination von Pulsdauer D und Intensität I in einem Parameterbereich PA auf, welche eine Photoablation bewirken (siehe Figur 4).

Wie in den Figuren 1 und 2 überdies schematisch dargestellt ist, umfasst die Vorrichtung 1 in einer Ausführungsvariante einen Luftfeuchtigkeitssensor 17 und einen Luftbefeuchter 16, die beispielsweise in einer geschlossenen Luftfeuchtekammer 160 angebracht sind. Der Luftfeuchtigkeitssensor 17 und der Luftbefeuchter 16 sind über eine Signalleitung 171 respektive über eine Steuerleitung 161 mit der Steuereinrichtung 14 respektive mit dessen elektronischen Schaltkreis 15 verbunden. Der elektronische Schaltkreis 15 ist als programmierter Prozessor, als applikationsspezifische integrierte Schaltung (ASIC) oder als andere elektronische Logikeinheit ausgeführt.

Der elektronische Schaltkreis 15 ermittelt über die Signalleitung 171 die vom Luftfeuchtigkeitssensor 17 gemessene relative Luftfeuchtigkeit im Umgebungsbereich U des zu bearbeitenden Implantationsmaterials 2. Der elektronische Schaltkreis 15 ist eingerichtet, den Luftbefeuchter 16 abhängig vom gemessenen Luftfeuchtigkeitswert derart zu steuern, dass ein vorgegebener Mindestluftfeuchtigkeitswert eingehalten wird. Ein Wassertank und/oder eine Wasserleitung zur Zuführung von Wasser zum Luftbefeuchter 16 ist in den Figuren 1 und 2 nicht dargestellt. Der Mindestluftfeuchtigkeitswert beträgt beispielsweise mindestens 90% relative Feuchtigkeit, insbesondere 95% relative Feuchtigkeit. Die in den Figuren 1 und 2 schematisch dargestellte optionale Luftfeuchtekammer 160 vereinfacht und erhöht die Genauigkeit der einzuhaltenden relativen Luftfeuchtigkeit.

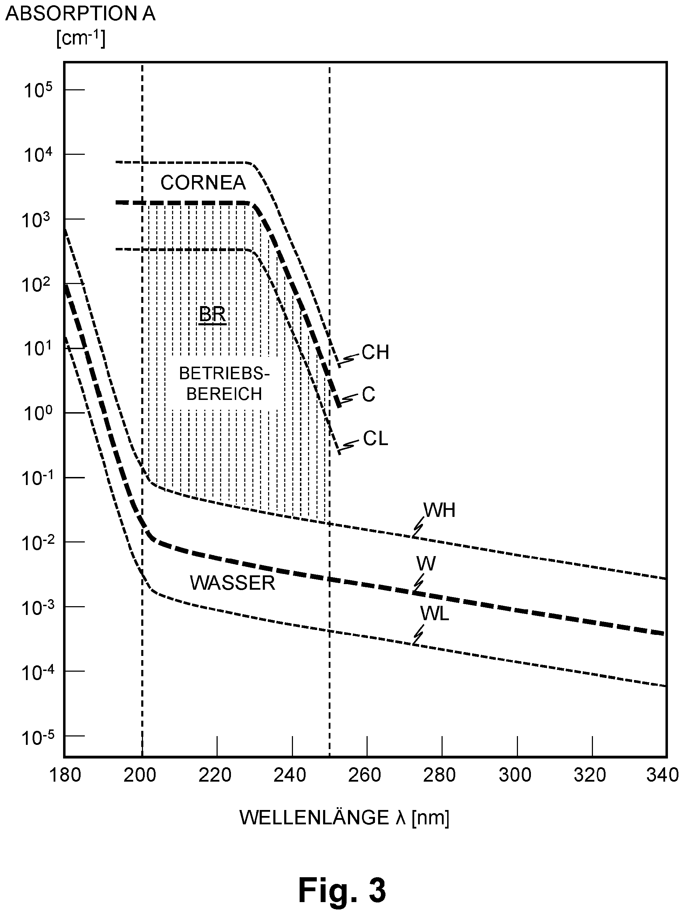

Die Laserquelle 11 ist eingerichtet einen gepulsten Laserstrahl L mit einer Wellenlänge λ im Ultraviolett-Wellenbereich zu erzeugen, wobei die Wellenlänge λ grösser als 193nm ist. Die Laserquelle 11 ist zudem eingerichtet den gepulsten Laserstrahl L mit einer Wellenlänge λ in einem Wellenlängenbereich zu erzeugen, in welchem die Wellenlänge λ im Grundmaterial des Implantationsmaterials 2 einen höheren Absorptionsgrad A des gepulsten Laserstrahls L bewirkt als im Wasser des Implantationsmaterials 2, z.B. in einem Betriebsbereich BB gemäss Figur 3. Die Laserquelle 11 ist somit eingerichtet den gepulsten Laserstrahl L mit einer Bearbeitungswellenlänge λ zu erzeugen, die einerseits über 193nm liegt, also über der Wellenlänge bekannter ArF Excimer Laser, und andererseits im Grundmaterial des Implantationsmaterials 2 einen höheren Absorptionsgrad A aufweist als im Wasser.

Dieser Zusammenhang von Wellenlänge λ und Absorptionsgrad A im Grundmaterial des Implantationsmaterials 2 einerseits und im Wasser andererseits ist in Figur 3 dargestellt. Figur 3 illustriert abhängig von der Wellenlänge λ des Laserstrahls L die Absorption A von Laserlicht in Wasser und in Protein, als Beispiel von Grundmaterial der Cornea. Der Verlauf des Absorptionsgrads A abhängig von der Wellenlänge λ ist für Wasser mit den Kurven WH, W und WL dargestellt. Dabei illustrieren die wellenlängenabhängigen Absorptionskurven WH und WL für Wasser einen Wertebereich mit hoher respektive tiefer Absorptionsrate von Licht im Wasser. Die wellenlängenabhängige Absorptionskurve W für Wasser entspricht einer mittleren Absorptionsrate von Licht im Wasser. Die unterschiedlichen wellenlängenabhängigen Absorptionskurven WH, W und WL sind einerseits durch verschiedene Reinheitsgrade des Wassers und unterschiedliche Messbedingungen begründet. Der Verlauf des Absorptionsgrads A abhängig von der Wellenlänge λ ist für Protein als Grundmaterial der Cornea mit den Kurven CH, C und CL dargestellt. Dabei illustrieren die wellenlängenabhängigen Absorptionskurven CH und CL für Protein (Cornea) einen Wertebereich mit hoher respektive tiefer Absorptionsrate von Licht im Protein (Cornea). Die wellenlängenabhängige Absorptionskurve C für Protein (Cornea) entspricht einer mittleren Absorptionsrate von Licht im Protein (Cornea). Die unterschiedlichen wellenlängenabhängigen Absorptionskurven CH, C und CL sind insbesondere durch unterschiedliche Messbedingungen begründet.

Das Bezugszeichen BB bezeichnet in der Figur 3 den Betriebsbereich für die Laserquelle 11 der Vorrichtung 1. Wie in der Figur 3 illustriert ist, ist der Betriebsbereich BB einerseits durch den Verlauf der wellenlängenabhängigen Absorptionskurven WH, W und WL für Wasser und andererseits durch die wellenlängenabhängigen Absorptionskurven CH, C und CL für Protein (Cornea) bestimmt, beispielsweise durch die mittlere wellenlängenabhängige Absorptionskurve W für Wasser und die mittlere wellenlängenabhängige Absorptionskurve C für Protein (Cornea). Dabei ist der Betriebsbereich BB für die Laserquelle 11 so bestimmt, dass die von der Wellenlänge λ abhängige Absorption A des gepulsten Laserstrahls L im Protein (Cornea) stets grösser ist als im Wasser. Im unteren Wellenlängenbereich, bei ca. 200nm, wird der Betriebsbereich BB durch den steilen Anstieg der Absorptionsrate A im Wasser für darunterliegende Wellenlängen begrenzt (in dem mit BL bezeichneten Bereich). Im oberen Wellenlängenbereich wird der Betriebsbereich BB durch den Abfall der Absorptionsrate A im Protein (Cornea) bei ca. 250nm begrenzt. Dort nähert sich die Absorptionsrate CL im Protein (Cornea) derart an die Absorptionsrate im Wasser WH an, dass im Extremfall (in dem mit BH bezeichneten Bereich), wenn die wellenlängenabhängige Absorptionskurve WH mit hoher Absorptionsrate im Wasser und die wellenlängenabhängige Absorptionskurve CL mit tiefer Absorptionsrate CL im Protein (Cornea) berücksichtigt werden, der Unterschied in den Absorptionsraten im Wasser WH und im Protein (Cornea) CL auf einen Faktor unter 10² fällt und sich an Faktor 10¹ annähert.

In der Figur 1 ist schematisch ein Ausführungsbeispiel dargestellt, in welchem eine Scannervorrichtung 13 mit einem oder mehreren beweglichen Spiegel 131 zum Ablenken des gepulsten Laserstrahls L, der Projektionsoptik 12 nachgeschaltet ist. In diesem Fall wird der gepulste Laserstrahl L durch die Projektionsoptik 12 für die Bestrahlung der Oberfläche 20 in einem Bearbeitungsbereich 21 des zu behandelnden Implantationsmaterial 2 aufbereitet (Fokussierung/Konvergierung). Der Bearbeitungsbereich 21 wird durch die Scannervorrichtung 13 durch Ablenkung des gepulsten Laserstrahls L mittels eines oder mehrerer beweglicher Spiegel 131 gemäss einem Bearbeitungsmuster bewegt, beispielsweise zur Erzeugung einer Lentikeloberfläche eines aus dem Implantationsmaterial 2 zu erzeugenden Lentikels.

In der Figur 2 ist schematisch ein Ausführungsbeispiel dargestellt, in welchem die Scannervorrichtung 13 mit einem oder mehreren beweglichen Spiegel 131 der Projektionsoptik 12 vorgeschaltet ist.

In weiteren Ausführungsvarianten umfasst die Scannervorrichtung 13 einen oder mehrere Antriebe 132 zum Verschieben der Projektionsoptik 12, um die Bewegung des Bearbeitungsbereichs 21 gemäss dem Bearbeitungsmuster auszuführen.

Sowohl in der Figur 1 als auch in der Figur 2 ist schematisch eine weitere alternative Ausführungsvariante der Scannervorrichtung 13' illustriert. Die Scannervorrichtung 13' umfasst einen oder mehrere Antriebe 132' umfasst, die eingerichtet sind, einen Materialträger 131' zu verschieben, auf dem das zu bearbeitende Implantationsmaterial 2 angebracht ist, um die Bewegung des Bearbeitungsbereichs 21 gemäss dem Bearbeitungsmuster auszuführen.

## Patentansprüche

1. Vorrichtung (1) zur Ablationsbearbeitung von ophthalmologischem Implantationsmaterial (2), welches durch wasserhaltiges Grundmaterial gebildet ist, umfassend:
eine Laserquelle (11), die eingerichtet ist, einen gepulsten Laserstrahl (L) mit einer Bearbeitungswellenlänge (λ) im Ultraviolett-Wellenbereich zu erzeugen, wobei die Bearbeitungswellenlänge (λ) in einem Wellenlängenbereich von 200nm bis 250nm liegt und im Grundmaterial des Implantationsmaterials (2) einen höheren Absorptionsgrad (A) des Laserstrahls (L) bewirkt als der Absorptionsgrad (A) des Laserstrahls (L) im Wasser des Implantationsmaterials (2),
eine Projektionsoptik (12), die eingerichtet ist, den gepulsten Laserstrahl (L) auf eine Oberfläche (20) des Implantationsmaterials (2) zu strahlen, und in einem Bearbeitungsbereich (21) mit Laserpulsen (P) des Laserstrahls (L), welche Laserpulse (P) eine photoablationbewirkende Kombination von Pulsdauer (D) und Intensität (I) aufweisen, wobei die Intensität (I) der Laserpulse (P) in einem Intensitätsbereich von 10⁶ W/cm² bis 10¹¹ W/cm² liegt, eine Interaktion mit dem Implantationsmaterial (2) zur Ablation des Implantationsmaterials (2) auszulösen, und
eine Scannervorrichtung (13, 13'), die eingerichtet ist, eine Bewegung des Bearbeitungsbereichs (21) zur Ablationsbearbeitung gemäss einem Bearbeitungsmuster auszuführen.

2. Vorrichtung (1) nach Anspruch 1, **dadurch gekennzeichnet, dass** die Laserquelle (11) eingerichtet ist, den gepulsten Laserstrahl (L) mit einer Bearbeitungswellenlänge (λ) in einem Wellenlängenbereich zu erzeugen, welcher im tieferen Wellenlängenbereich durch einen maximal zu erreichenden Absorptionsgrad (A) von 10⁻²/cm des Laserstrahls (L) im Wasser des Implantationsmaterials (2) begrenzt ist und welcher im höheren Wellenlängenbereich durch einen minimal zu erreichenden Absorptionsgrad (A) von 10⁰/cm des Laserstrahls (L) im Grundmaterial des Implantationsmaterials (2) begrenzt ist.

3. Vorrichtung (1) nach einem der Ansprüche 1 bis 2, **dadurch gekennzeichnet, dass** die Pulsdauer (D) der Laserpulse (P) in einem Pulsdauerbereich von 10⁻¹⁰Sekunden bis 10⁻⁵ Sekunden liegt, insbesondere in einem Pulsdauerbereich von 10⁻⁹Sekunden bis 10⁻⁶ Sekunden.

4. Vorrichtung (1) nach einem der Ansprüche 1 bis 3, **dadurch gekennzeichnet, dass** die Intensität (I) der Laserpulse (P) in einem Intensitätsbereich von 10⁷W/cm² bis 10¹⁰W/cm² liegt.

5. Vorrichtung (1) nach einem der Ansprüche 1 bis 4, **dadurch gekennzeichnet, dass** die Laserquelle (11) und die Projektionsoptik (12) eingerichtet sind, den gepulsten Laserstrahl (L) mit einer Fluenz im Fluenzbereich von 10⁶W/cm² und 10¹⁰W/cm² auf die Oberfläche (20) des Implantationsmaterials (2) zu strahlen.

6. Vorrichtung (1) nach einem der Ansprüche 1 bis 5, **dadurch gekennzeichnet, dass** die Vorrichtung (1) einen Luftbefeuchter (16), einen Luftfeuchtigkeitssensor (17) und eine mit dem Luftbefeuchter (16) und dem Luftfeuchtigkeitssensor (17) verbundene Steuereinrichtung (14) umfasst, wobei die Steuereinrichtung (14) einen elektronischen Schaltkreis (15) umfasst, der eingerichtet ist, den Luftbefeuchter (16) abhängig von einem vom Luftfeuchtigkeitssensor (17) in einem an das Implantationsmaterial (2) angrenzenden Umgebungsbereich (U) gemessenen Luftfeuchtigkeitswert derart zu steuern, dass ein vorgegebener Mindestluftfeuchtigkeitswert eingehalten wird.

7. Vorrichtung (1) nach Anspruch 6, **dadurch gekennzeichnet, dass** der elektronische Schaltkreis (15) eingerichtet ist, den Luftbefeuchter (16) derart zu steuern, dass ein Mindestluftfeuchtigkeitswert von 90% relativer Feuchtigkeit eingehalten wird, insbesondere ein Mindestluftfeuchtigkeitswert von 95% relativer Feuchtigkeit.

8. Vorrichtung (1) nach einem der Ansprüche 1 bis 7, **dadurch gekennzeichnet, dass** die Scannervorrichtung (13, 13') eingerichtet ist, die Bewegung des Bearbeitungsbereichs (21) zur Ablationsbearbeitung gemäss einem Bearbeitungsmuster zur Erzeugung einer Lentikeloberfläche auszuführen.

9. Vorrichtung (1) nach einem der Ansprüche 1 bis 8, **dadurch gekennzeichnet, dass** die Scannervorrichtung (13) mindestens einen beweglichen Spiegel (131) umfasst, der eingerichtet ist, den gepulsten Laserstrahl (L) zur Bewegung des Bearbeitungsbereichs (21) gemäss dem Bearbeitungsmuster abzulenken.

10. Vorrichtung (1) nach Anspruch 9, **dadurch gekennzeichnet, dass** die Scannervorrichtung (13) der Projektionsoptik (12) nachgeschaltet angeordnet ist.

11. Vorrichtung (1) nach einem der Ansprüche 1 bis 9, **dadurch gekennzeichnet, dass** die Scannervorrichtung (13) mindestens einen Antrieb (132) umfasst, der eingerichtet ist, die Projektionsoptik (12) zu verschieben, um die Bewegung des Bearbeitungsbereichs (21) gemäss dem Bearbeitungsmuster auszuführen.

12. Vorrichtung (1) nach einem der Ansprüche 1 bis 9, **dadurch gekennzeichnet, dass** die Scannervorrichtung (13') mindestens einen Antrieb (132') umfasst, der eingerichtet ist, einen Materialträger (131') zu verschieben, auf dem das Implantationsmaterial (2) angebracht ist, um die Bewegung des Bearbeitungsbereichs (21) gemäss dem Bearbeitungsmuster auszuführen.

## Claims

1. A device (1) for ablation processing of ophthalmic implantation material (2) formed by aqueous base material, comprising:
a laser source (11) configured to generate a pulsed laser beam (L) having a processing wavelength (λ) in the ultraviolet waveband, the processing wavelength (λ) being in a wavelength range of 200nm to 250nm and causing a higher absorbance (A) of the laser beam (L) in the base material of the implantation material (2) than the absorbance (A) of the laser beam (L) in the water of the implantation material (2),
a projection optics (12) configured to radiate the pulsed laser beam (L) onto a surface (20) of the implantation material (2), and in a processing area (21) with laser pulses (P) of the laser beam (L) which laser pulses (P) have a photoablation-effective combination of pulse duration (D) and intensity (I), the intensity (I) of the laser pulses (P) being in an intensity range of 10⁶ W/cm² to 10¹¹ W/cm², to trigger interaction with the implantation material (2) for ablation of the implantation material (2), and
a scanner device (13, 13') configured to perform movement of the processing area (21) for ablation processing according to a processing pattern.

2. The device (1) according to claim 1, **characterized in that** the laser source (11) is configured to generate the pulsed laser beam (L) with a processing wavelength (λ) in a wavelength range which in the lower wavelength range is limited by a maximum absorbance (A) to be achieved of 10⁻²/cm of the laser beam (L) in the water of the implantation material (2) and which in the higher wavelength range is limited by a minimum absorbance (A) to be achieved of 10⁰/cm of the laser beam (L) in the base material of the implantation material (2).

3. Device (1) according to one of claims 1 to 2, **characterized in that** the pulse duration (D) of the laser pulses (P) is in a pulse duration range of 10⁻¹⁰ seconds to 10⁻⁵ seconds, in particular in a pulse duration range of 10⁻⁹ seconds to 10⁻⁶ seconds.

4. Device (1) according to any one of claims 1 to 3, **characterized in that** the intensity (I) of the laser pulses (P) is in an intensity range from 10⁷ W/cm² to 10¹⁰ W/cm².

5. Device (1) according to any one of claims 1 to 4, **characterized in that** the laser source (11) and the projection optics (12) are configured to radiate the pulsed laser beam (L) with a fluence in the fluence range of 10⁶ W/cm² and 10¹⁰ W/cm² onto the surface (20) of the implantation material (2).

6. Device (1) according to any one of claims 1 to 5, **characterized in that** the device (1) comprises a humidifier (16), an air humidity sensor (17), and a controller (14) connected to the humidifier (16) and the air humidity sensor (17), the controller (14) comprising an electronic circuit (15) which is configured to control the humidifier (16) as a function of an air humidity value measured by the air humidity sensor (17) in an ambient area (U) adjacent to the implantation material (2) in such a way that a predetermined minimum air humidity value is maintained.

7. Device (1) according to claim 6, **characterized in that** the electronic circuit (15) is configured to control the humidifier (16) in such a way that a minimum air humidity value of 90% relative humidity is maintained, in particular a minimum air humidity value of 95% relative humidity.

8. Device (1) according to any one of claims 1 to 7, **characterized in that** the scanner device (13, 13') is configured to execute the movement of the processing area (21) for ablation processing according to a processing pattern for generating a lenticular surface.

9. Device (1) according to any one of claims 1 to 8, **characterized in that** the scanner device (13) comprises at least one movable mirror (131) configured to deflect the pulsed laser beam (L) for moving the processing area (21) according to the processing pattern.

10. Device (1) according to claim 9, **characterized in that** the scanner device (13) is arranged downstream of the projection optics (12).

11. Device (1) according to any one of claims 1 to 9, **characterized in that** the scanner device (13) comprises at least one drive (1 32) configured to displace the projection optics (1 2) in order to carry out the movement of the processing area (21) according to the processing pattern.

12. Device (1) according to any one of claims 1 to 9, **characterized in that** the scanner device (13') comprises at least one drive (132') configured to displace a material carrier (131') on which the implantation material (2) is mounted to perform the movement of the processing area (21) according to the processing pattern.

## Revendications

1. Dispositif (1) de traitement d'ablation de matériau d'implantation ophtalmique (2) formé par un matériau de base aqueux, comprenant:
une source laser (11) qui est conçue pour générer un faisceau laser pulsé (L) ayant une longueur d'onde de traitement (λ) dans la gamme d'ondes ultraviolettes, la longueur d'onde de traitement (λ) étant située dans une gamme de longueurs d'onde de 200 nm à 250 nm et provoquant dans le matériau de base du matériau d'implantation (2) un degré d'absorption (A) du faisceau laser (L) supérieur au degré d'absorption (A) du faisceau laser (L) dans l'eau du matériau d'implantation (2),
une optique de projection (12) qui est conçue pour projeter le faisceau laser pulsé (L) sur une surface (20) du matériau d'implantation (2), et dans une zone de traitement (21) avec des impulsions laser (P) du faisceau laser (L), lesquelles impulsions laser (P) présentent une combinaison de durée d'impulsion (D) et d'intensité (I) agissant par photoablation, l'intensité (I) des impulsions laser (P) étant située dans une plage d'intensité de 10⁶ W/cm² à 10¹¹ W/cm², à déclencher une interaction avec le matériau d'implantation (2) pour l'ablation du matériau d'implantation (2), et
un dispositif de balayage (13, 13') qui est conçu pour exécuter un mouvement de la zone de traitement (21) pour le traitement d'ablation selon un motif de traitement.

2. Dispositif (1) selon la revendication 1, **caractérisé en ce que** la source laser (11) est conçue pour générer le faisceau laser pulsé (L) avec une longueur d'onde de traitement (λ) dans une gamme de longueurs d'onde, qui est limitée dans la gamme de longueurs d'onde inférieure par un degré d'absorption (A) maximal à atteindre de 10⁻²/cm du faisceau laser (L) dans l'eau du matériau d'implantation (2) et qui est limitée dans la gamme de longueurs d'onde supérieure par un degré d'absorption (A) minimal à atteindre de 10⁰/cm du faisceau laser (L) dans le matériau de base du matériau d'implantation (2).

3. Dispositif (1) selon l'une des revendications 1 à 2, **caractérisé en ce que** la durée d'impulsion (D) des impulsions laser (P) est comprise dans une plage de durée d'impulsion de 10⁻¹⁰ secondes à 10⁻⁵ secondes, notamment dans une plage de durée d'impulsion de 10⁻⁹ secondes à 10⁻⁶ secondes.

4. Dispositif (1) selon l'une des revendications 1 à 3, **caractérisé en ce que** l'intensité (I) des impulsions laser (P) est comprise dans une plage d'intensité de 10⁷ W/cm² à 10¹⁰ W/cm².

5. Dispositif (1) selon l'une des revendications 1 à 4, **caractérisé en ce que** la source laser (11) et l'optique de projection (12) sont conçues pour projeter le faisceau laser pulsé (L) sur la surface (20) du matériau d'implantation (2) avec une fluence comprise dans la plage defluence 10⁶ W/cm² et 10¹⁰ W/cm².

6. Dispositif (1) selon l'une des revendications 1 à 5, **caractérisé en ce que** le dispositif (1) comprend un humidificateur (16), un capteur d'humidité (17) et un dispositif de commande (14) relié à l'humidificateur (16) et au capteur d'humidité (17), le dispositif de commande (14) comprenant un circuit électronique (15), qui est conçu pour commander l'humidificateur (16) en fonction d'une valeur d'humidité de l'air mesurée par le capteur d'humidité (17) dans une zone d'environnement (U) adjacente au matériau d'implantation (2), de manière à respecter une valeur d'humidité minimale prédéterminée.

7. Dispositif (1) selon la revendication 6, **caractérisé en ce que** le circuit électronique (15) est conçu pour commander l'humidificateur (16) de manière à respecter une valeur d'humidité minimale de 90% d'humidité relative, notamment une valeur d'humidité minimale de 95% d'humidité relative.

8. Dispositif (1) selon l'une des revendications 1 à 7, **caractérisé en ce que** le dispositif de balayage (13, 13') est conçu pour effectuer le déplacement de la zone de traitement (21) pour le traitement d'ablation selon un motif de traitement pour générer une surface lenticulaire.

9. Dispositif (1) selon l'une des revendications 1 à 8, **caractérisé en ce que** le dispositif de balayage (13) comprend au moins un miroir mobile (131) qui est conçu pour dévier le faisceau laser pulsé (L) pour déplacer la zone de traitement (21) selon le motif de traitement.

10. Dispositif (1) selon la revendication 9, **caractérisé en ce que** le dispositif de balayage (13) est disposé en aval de l'optique de projection (12).

11. Dispositif (1) selon l'une des revendications 1 à 9, **caractérisé en ce que** le dispositif de balayage (13) comprend au moins un actionneur (132) qui est conçu pour déplacer l'optique de projection (12) afin d'effectuer le déplacement de la zone de traitement (21) selon le motif de traitement.

12. Dispositif (1) selon l'une des revendications 1 à 9, **caractérisé en ce que** le dispositif de balayage (13') comprend au moins un actionneur (132') conçu pour déplacer un support de matériau (131') sur lequel le matériau d'implantation (2) est appliqué, afin d'effectuer le déplacement de la zone de traitement (21) selon le motif de traitement.
